(19) Europäisches Patentamt
European Patent Office
Office européen des brevets

(11) **EP 4 744 591 A1**

(12) **EUROPEAN PATENT APPLICATION**

(43) Date of publication:
20.05.2026 Bulletin 2026/21

(51) International Patent Classification (IPC):
*A61B 6/03* (2006.01)        *A61B 6/00* (2024.01)

(21) Application number: 24213024.3

(22) Date of filing: 14.11.2024

(52) Cooperative Patent Classification (CPC):
A61B 6/482; A61B 6/032; A61B 6/5205;
A61B 6/5217

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Koninklijke Philips N.V.
5656 AE Eindhoven (NL)

(72) Inventors:
• ROMMAN, Zimam
  Eindhoven (NL)
• SEGEV-STEINBUCH, Lior
  Eindhoven (NL)

(74) Representative: Philips Intellectual Property &
Standards
High Tech Campus 52
5656 AG Eindhoven (NL)

(54) **A METHOD AND SYSTEM FOR GENERATING A MEDICAL IMAGE INDICATING THE PRESENCE OF URIC ACID**

(57) A method is provided for generating a medical image indicating the presence of uric acid. A base image indicates the presence of uric acid on a pixel-by-pixel basis. Pixel values of the base image are modified using the effective atomic number and the electron density values associated for the tissue associated with those pixels.

FIG. 3

## Description

FIELD OF THE INVENTION

This invention relates to the detection of uric acid (UA), in particular by detection of monosodium urate in medical images, which is one of the more common salts formed by uric acid ions.

BACKGROUND OF THE INVENTION

Uric acid is continually produced as a byproduct of purine metabolism. Ordinarily, uric acid is excreted either through the kidneys (via active secretion) or the gastro-intestinal tract. Gout is a crystal-induced arthropathy, caused by monosodium urate crystals.

It is known to detect the presence of monosodium urate crystals using a spectral UA image (or map). This image is one of the spectral results which are currently generated by known spectral CT scanners. The spectral UA result is meant to aid in the diagnosis of gout disease, in which monosodium urate crystals are accumulated within the joint and cause arthritis. The spectral UA map also allows radiologists to detect the presence of monosodium stones which may be present in abdominal-pelvic organs, and cause inflammation and obstruction.

The current state of the uric acid image does not allow radiologists to diagnose gout cases with high diagnostic confidence due to a high level of false positives and a very low specificity evident in the image. Testimonies made by radiologists indicate that customers have even stopped using UA images for the diagnosis of gout.

There is therefore a need for an improvement to the uric acid map in order to reduce the number of false positives and increase the specificity of the spectral result. This would increase the diagnostic confidence of gout using spectral UA images.

SUMMARY OF THE INVENTION

The invention is defined by the claims.

According to examples in accordance with an aspect of the invention, there is provided a method of generating a medical image indicating the presence of uric acid, comprising:

receiving a uric acid image of tissue as a base image which indicates the presence of uric acid on a pixel-by-pixel basis;
receiving an effective atomic number for tissue corresponding to pixels of the base image;
receiving an electron density value for tissue corresponding to the pixels of the base image; and
modifying pixel values of the base image using the associated effective atomic number and the electron density value.

The method improves existing types of uric acid (UA) image. The modification reduces false positives, in particular by modifying pixels of the base image which indicate the presence of uric acid to reduce false positives. The modified image is to allow radiologists to detect the presence of monosodium urate crystals or stones. This enables screening for (i) gout disease, in which monosodium urate crystals precipitate within joints and soft tissue and cause arthritis and (ii) UA stones which may be present in abdominal-pelvic organs, cause inflammation and obstruction.

The method for example comprises generating the uric acid image by processing low energy data and high energy data of a dual layer CT image to derive Compton-like data and photoelectric-like data. This is a known way to process a dual layer CT image to enable spectral basis images to be derived.

The effective atomic number and electron density values are preferably received only for each pixel of the base image for which the presence of uric acid is indicated. In this way, the processing requirements are reduced.

The modifying for example comprises:

testing if the obtained values of the effective atomic number and the electron density meet a threshold condition; and
if the threshold condition is met, modifying the pixel information such that the presence of uric acid is no longer indicated.

In this way, if the effective atomic number and electron density values together are indicative of a false positive, the pixel information is modified to remove the false positive.

The testing for example comprises:

deriving a test value:

$$Zeff * ZeffSlope + ED * EDSlope,$$

where Zeff is the effective atomic number, ED is the electron density, and ZeffSlope and EDSlope are multipliers; and
testing if the value is below a threshold, wherein if the value is below the threshold, the threshold condition is met.

This disclosure also provides a computer program comprising computer program code which is adapted, when said program is run on a computer, to perform the method defined above.

This disclosure also provides an image processing system comprising:
a processor which is configured to

receive a uric acid image of tissue as a base image which indicates the presence of uric acid on a pixel-by-pixel basis;

receive an effective atomic number for tissue corresponding to pixels of the base image;
receive an electron density value for tissue corresponding to the pixels of the base image; and
modify the pixel values of the base image using the associated effective atomic number and the electron density value.

The uric acid image for example comprises a dual layer CT image in which low energy data and high energy data are processed to derived Compton-like data and photoelectric-like data.

The method preferably comprises receiving the effective atomic number and electron density values only for each pixel of the base image for which the presence of uric acid is detected.

The processor is for example configured to modify the pixels by:

testing if the obtained values of the effective atomic number and the electron density meet a threshold condition; and
if the threshold condition is met, modifying the pixel information such that the presence of uric acid is no longer indicated.

The processor is for example configured to perform the testing by:

deriving a test value:

$$Zeff * ZeffSlope + ED * EDSlope,$$

where Zeff is the effective atomic number, ED is the electron density, and ZeffSlope and EDSlope are multipliers; and
testing if the value is below a threshold, wherein if the value is below the threshold, the threshold condition is met.

This disclosure also provides a medical imaging system comprising:

a CT scanner for generating a uric acid image of tissue as a base image which indicates the presence of uric acid on a pixel-by-pixel basis; and
the image processing system define above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows a method of generating a medical image indicating the presence of uric acid;
Fig. 2 shows in more detail the final steps in generating the medical image; and
Fig 3 shows a medical imaging system.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for generating a medical image indicating the presence of uric acid. A base image indicates the presence of uric acid on a pixel-by-pixel basis. Pixel values of the base image are modified (so that they no longer indicate the presence) using the effective atomic number and the electron density values for the tissue associated with those pixels.

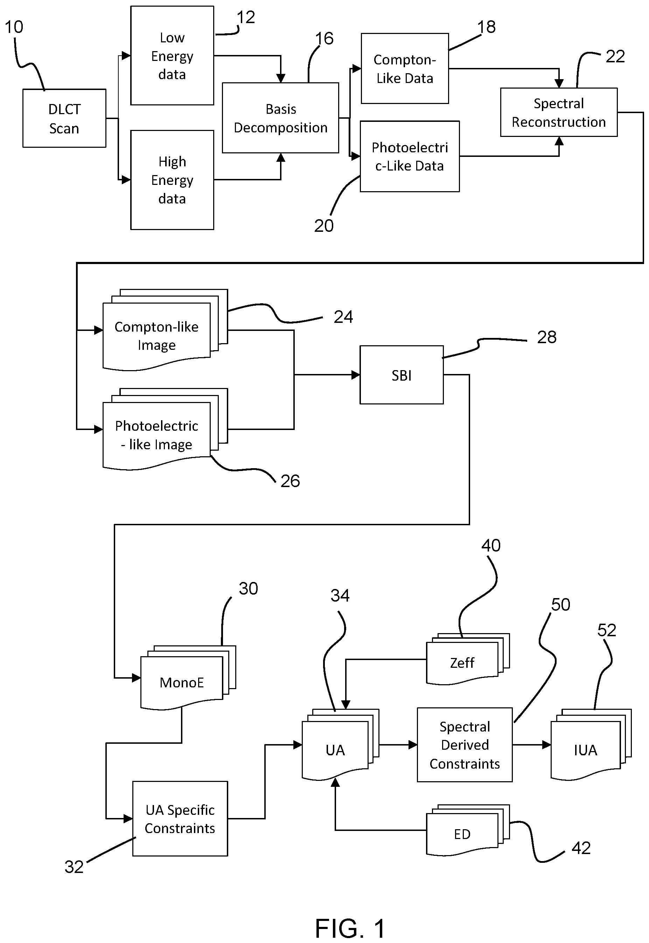

Fig. 1 shows a method of generating a medical image indicating the presence of uric acid.

In step 10, a dual layer CT (DLCT) scan is performed. The dual layer scanner has a top layer, in particular an yttrium-based garnet scintillator, for absorbing low energy photons and a bottom layer, in particular formed of gadolinium-oxysulphide, for absorbing high energy photons.

This yields low energy data 12 and high energy data 14. They are combined using basis decomposition in step 16. This is a projection-space decomposition to derive a pair of basis functions, wherein a first function approximates Compton scattering to generate Compton-like data 18 and a second function approximates the photoelectric effect to generated photoelectric-like data 20. Spectral reconstruction in step 22 yields a Compton-like image 24 and a photoelectric-like image 26 as basis pair images. Together, they form a spectral base image 28.

A mono energetic (MonoE) spectral image 30 is derived a linear combination of the basis pair images 24, 26

Constraints are applied in step 32 to the mono energetic spectral image 30. In particular, thresholds are applied to the image derived from uric acid behavior in the dual energy map. This results in a UA image 34.

The UA image represents the presence or not of uric acid, pixel-by pixel. The uric acid images are a known part of the spectral portfolio of many CT vendors.

In medical practice, these uric acid images are for example used to diagnose conditions such as gout, where uric acid crystals accumulate in joints or tissues, leading to inflammation and pain.

The steps described above are all known, and will accordingly not be elaborated further. By way of example, these steps are disclosed in the paper "Detector-based spectral CT with a novel dual-layer technology: principles and applications", Negin Rassouli et. al., Insights Imaging (2017) 8:589-598.

The resulting image 34 is thus the UA image generated using known processing.

This disclosure relates to an approach by which the uric acid image is improved with further processing, and in particular the number of false positives, i.e. the pixels that indicate the presence of uric acid when it is not present, is reduced.

In step 40, an effective atomic number, Zeff, is obtained on a per-pixel basis. As described in Rassouli et. al., the effective atomic number is derived from the ratio of the photoelectric component coefficient and the Compton scatter component coefficient. This enables the material characteristics at each imaging pixel to be obtained. The effective atomic number is in essence obtained by analyzing the attenuation of X-rays at different energy levels or wavelengths and determining the composition of the imaged materials based on their X-ray absorption characteristics.

The effective atomic number is a weighted average of the atomic numbers of the elements present in a material, taking into account their relative proportions and X-ray interaction cross-sections. It is know to derive the effective atomic number information in order to provide additional insights into tissue composition, density, or the presence of specific materials, which can be useful for diagnostic purposes.

In step 42, an electron density is obtained. The determination of electron density during dual energy CT imaging is known, for example as described in the paper "Accuracy of electron density, effective atomic number, and Iodine concentration determination with a dual-layer dual-energy computed tomography system", Chia-ho Jua et. al., Med. Phys. 2108 June 45(6): 2486-2497.

The electron density is obtained from a linear combination of the basis pair images 24, 26. A best fit to expected electron densities of literature tissues is used.

The electron density is generated using decomposition techniques derived from the dual energy basis materials. Electron density images have the potential for diagnostic use based on the mass density of materials.

Constraints are applied in step 50. In particular, true positive and false positive UA detections in the image 34 are separated using the Zeff and ED values. After the false positives are removed, the improved UA image 52 results.

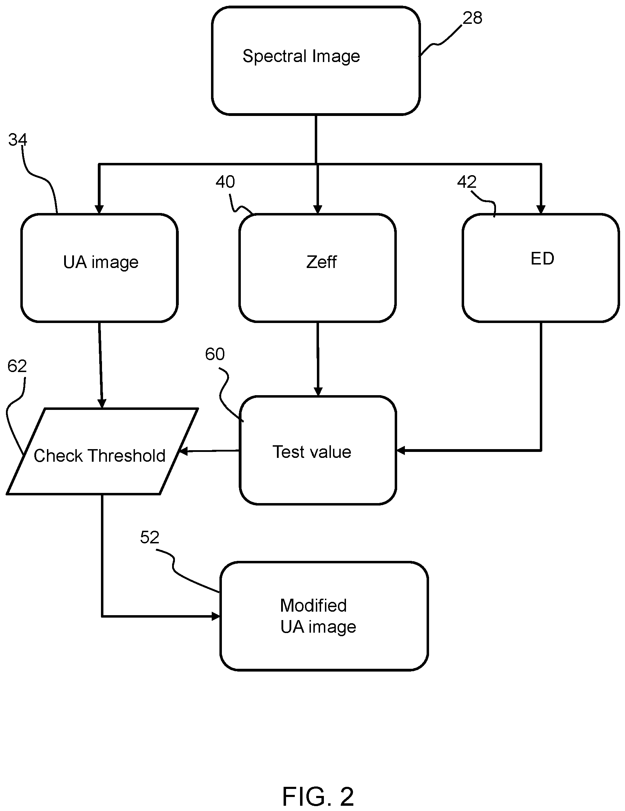

Fig. 2 shows the final steps in more detail.

In step 28, the spectral base image is obtained from the medical imaging system, as explained above. The imaging system is for example a CT (computed tomography) imager, or a spectral CT imager.

In step 34, the known uric acid image is derived as explained above. The Zeff and ED values are obtained in steps 40 and 42 as explained above.

In a preferred example, the effective atomic number and electron density values are received only for those pixels of the spectral base image 28 for which the presence of uric acid is detected in the UA image 34.

A test value is derived from the effective atomic number and electron density value in step 60. This test value is used to test if the tissue at the location corresponding to a particular pixel has falsely been identified as showing the presence of uric acid.

The test value in one example is:

$$Zeff * ZeffSlope + ED * EDSlope$$

This is a linear combination of the Zeff and ED values using slope values ZeffSlope and EDSlope as multipliers.

The slope values are derived from analysis of clinical or phantom scans. For example, uric acid regions (gout regions) of previous scans are analyzed, whereby each pixel with the UA region is expressed by the ED and Zeff values. Using a scatter plot, the slope values can be set which provide a boundary to the scatter points, hence separating valid pairs of Zeff and ED values from invalid pairs. Thus, the slope values are values obtained by previous analysis. The slope values may be considered to be obtained as part of a system (factory) calibration procedure.

It has been found by analyzing UA crystals that both electron density and Zeff values are relatively low.

The values are checked against a threshold Th in step 18. Any pixel that obtains a value below the threshold is then nulled in the UA image 12. In other words, a pixel that indicated presence of UA is modified so that it no longer indicates the presence of UA. The resultant modified UA image will then only include pixels which were originally present in the UA image 34, and are above the threshold.

Thus, a false positive is identified as:

$$Zeff * ZeffSlope + ED * EDSlope <= Th$$

In this equation, Zeff is unitless. The electron density value is relative to the electron density of water and hence is also unitless. The multipliers ZeffSlope and EDSlope thus also dimensionless.

The linear separation line has the form:

$$Zeff = \alpha - \beta * ED$$

where $\alpha$ = Th/ZeffSlope and $\beta$= (EDSlope)/ZeffSlope

This defines a linear plot in the space [Zeff, ED], and the line separates false positives from true positives. The false positives are thus removed by testing with respect to a linear separation line.

The modified image reduces the false positive rate. The improved images, in the same way as current UA images, may be reconstructed on the scanner and sent to a picture archiving and communication system (PACS). As explained above, the images may be used for the identification of monosodium urate crystals accumulating in joints and soft tissues and causing arthritis.

The modified UA images have been shown to reduce false positive rates by more than 85%, resulting in an average <0.5 false positives per slice, in a series of analyzed studies. Sensitivity was shown to be maintained in all studies analyzed. The new images may increase the diagnostic confidence of spectral CT users.

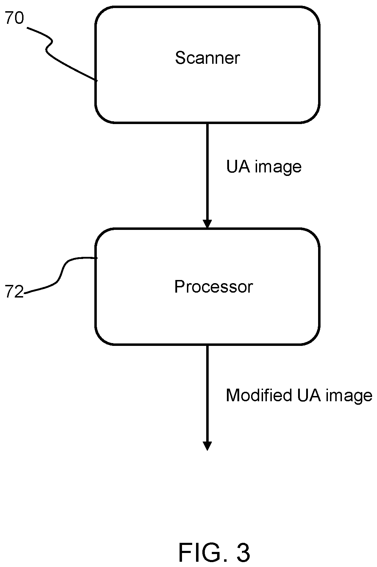

Fig. 3 shows a medical imaging system comprising a spectral CT scanner 70 and an image processing system 72. The scanner generates a uric acid image of tissue as a base image which indicates the presence of uric acid on a pixel-by-pixel basis. The image processing system comprises a processor which runs software to perform the image processing method explained above, thereby to generate the modified UA image.

Improving the UA image may increase the diagnostic confidence of not only UA images, but the entire bundle of spectral images, by eliminating a poor-quality spectral result.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A method of generating a medical image indicating the presence of uric acid, comprising:

    receiving a uric acid image (34) of tissue as a base image which indicates the presence of uric acid on a pixel-by-pixel basis;
    receiving an effective atomic number (40) for tissue corresponding to pixels of the base image;
    receiving an electron density value (42) for tissue corresponding to the pixels of the base image; and
    modifying (52) pixel values of the base image using the associated effective atomic number and the electron density value.

2. The method of claim 1, comprising generating the uric acid image by processing low energy data and high energy data of a dual layer CT image to derive Compton-like data and photoelectric-like data.

3. The method of claim 1 or 2, wherein the effective atomic number and electron density values are received only for each pixel of the base image for which the presence of uric acid is indicated.

4. The method of any one of claims 1 to 3, wherein the modifying comprises:

    (62) testing if the obtained values of the effective atomic number and the electron density meet a threshold condition; and
    (52) if the threshold condition is met, modifying the pixel information such that the presence of uric acid is no longer indicated.

5. The method of claim 4, wherein the testing comprises:
   deriving a test value:

    $Zeff * ZeffSlope + ED * EDSlope$, where $Zeff$ is the effective atomic number, $ED$ is the electron density, and $ZeffSlope$ and $EDSlope$ are multipliers; and
    testing if the value is below a threshold, wherein if the value is below the threshold, the threshold condition is met.

6. A computer program comprising computer program code which is adapted, when said program is run on a computer, to perform the method of any one of claims 1 to 5.

7. An image processing system comprising:
   a processor (72) which is configured to

   receive a uric acid image of tissue as a base image which indicates the presence of uric acid on a pixel-by-pixel basis;
   receive an effective atomic number for tissue corresponding to pixels of the base image;
   receive an electron density value for tissue corresponding to the pixels of the base image; and
   modify the pixel values of the base image using the associated effective atomic number and the electron density value.

8. The system of claim 7, wherein the uric acid image comprises a dual layer CT image in which low energy data and high energy data are processed to derived Compton-like data and photoelectric-like data.

9. The system of claim 7 or 8, comprising receiving the effective atomic number and electron density values only for each pixel of the base image for which the presence of uric acid is detected.

10. The system of any one of claims 7 to 9, wherein the processor is configured to modify the pixels by:

    testing if the obtained values of the effective atomic number and the electron density meet a threshold condition; and
    if the threshold condition is met, modifying the pixel information such that the presence of uric acid is no longer indicated.

11. The system of claim 10, wherein the processor is configured to perform the testing by:

    deriving a test value:

    $$Zeff * ZeffSlope + ED * EDSlope,$$

    where Zeff is the effective atomic number, ED is the electron density, and ZeffSlope and EDslope are multipliers; and
    testing if the value is below a threshold, wherein if the value is below the threshold, the threshold condition is met.

12. A medical imaging system comprising:

    a CT scanner for generating a uric acid image of tissue as a base image which indicates the presence of uric acid on a pixel-by-pixel basis; and
    the image processing system of any one of claims 7 to 11.

FIG. 1

FIG. 2

FIG. 3

Europäisches
Patentamt
European
Patent Office
Office européen
des brevets

**EUROPEAN SEARCH REPORT**

Application Number

**EP 24 21 3024**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 115 205 213 A (WEST CHINA HOSPITAL SICHUAN UNIV) 18 October 2022 (2022-10-18) * the whole document * ----- | 1-12 | INV. A61B6/03 A61B6/00 |
| A | CAROTTI MARINA ET AL: "The application of dual-energy computed tomography in the diagnosis of musculoskeletal disorders: a review of current concepts and applications", RADIOLOGIA MEDICA, SPRINGER ITALIA SRL, IT, vol. 124, no. 11, 4 March 2019 (2019-03-04), pages 1175-1183, XP036945131, ISSN: 0033-8362, DOI: 10.1007/S11547-019-01015-X [retrieved on 2019-03-04] * the whole document * ----- | 1-12 | |
| | | | TECHNICAL FIELDS SEARCHED (IPC) A61B |

The present search report has been drawn up for all claims

| Place of search | Date of completion of the search | Examiner |
|---|---|---|
| Munich | 10 March 2025 | Anscombe, Marcel |

EPO FORM 1503 03.82 (P4C01)

## ANNEX TO THE EUROPEAN SEARCH REPORT
## ON EUROPEAN PATENT APPLICATION NO.

EP 24 21 3024

This annex lists the patent family members relating to the patent documents cited in  the above-mentioned European search report.
The members are as contained in the European Patent Office EDP file on
The European Patent Office is in no way liable for these particulars which are merely given for the purpose of information.

10-03-2025

| Patent document cited in search report | Publication date | Patent family member(s) | Publication date |
|---|---|---|---|
| CN 115205213       A | 18-10-2022 | NONE | |

For more details about this annex : see Official Journal of the European Patent Office, No. 12/82

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **NEGIN RASSOULI**. Detector-based spectral CT with a novel dual-layer technology: principles and applications. *Insights Imaging*, 2017, vol. 8, 589-598

- **CHIA-HO JUA**. Accuracy of electron density, effective atomic number, and Iodine concentration determination with a dual-layer dual-energy computed tomography system. *Med. Phys.*, vol. 45 (6), 2486-2497